# EUROPEAN PATENT SPECIFICATION

(11) **EP 1 207 930 B1**
(45) Date of publication and mention of the grant of the patent: **22.06.2005**
(21) Application number: 00953841.4
(22) Date of filing: 04.08.2000
(51) Int. Cl.: A61M 25/00

(54) **REDUCED PROFILE DELIVERY SYSTEM**
EINFÜHRSYSTEM MIT REDUZIERTEM PROFIL
DISPOSITIF D'ADMINISTRATION A PROFIL REDUIT

(30) Priority: 06.08.1999 US 147645 P
(43) Date of publication of application: 29.05.2002
(73) Proprietor: Boston Scientific Scimed, Inc., Marple Grove, MN 55311-1566 (US)
(72) Inventor: GERDTS, Michael, D., Big Lake, MN 55343 (US)
(74) Representative: Sturt, Clifford Mark
(86) International application number: PCT/US2000/021388
(87) International publication number: WO 2001/010492

(56) References cited:
- EP-A- 0 732 117
- WO-A-96/33763
- US-A- 5 630 806
- US-A- 5 695 483

## Description

### Background of the Invention

The present invention relates to body insertable catheters and other tubular treatment devices, and more particularly to the manner in which such devices can be reinforced with metallic wires or strands.

Procedures involving entry into or treatment of body lumens, e.g., intravascular treatment procedures, frequently employ catheters. A variety of catheter types have been developed to meet different treatment needs. For example, U.S. Patent No. 4,547,193 discloses an angiographic catheter. U.S. Patent No. 5,364,357 discloses a small diameter coronary angioplasty catheter, more particularly a balloon dilatation catheter for expanding vascular passages. U.S. Patent No. 5,221,270 is drawn to a soft tip guiding catheter, which, after its insertion into a body lumen, is used to receive other devices and guide them through the same lumen. U.S. Patent No. 5,026,377 discloses a catheter used to deploy and position a self-expanding stent in a body lumen. U.S. Patent No. 5,695,483 discloses a catheter as defined in the preamble of claim 1 comprising an inner tubing member, a helically wound ribbon and an outer cover. The outer cover is shrank over the inner tubing member and helically wound ribbon so that it directly contacts the inner tubing member and helically wound ribbon.

The variety of uses notwithstanding, certain properties are necessary or desirable, regardless of the catheter type. All catheters must be biocompatible, with vascular catheters further requiring hemocompatibility. To varying degrees, catheters require sufficient axial strength to facilitate movement of the catheter distal end through vascular passages or other body lumens by applying an axial pushing force near the catheter proximal end. This characteristic is often called "pushability". A related characteristic, "torqueability", refers to the ability to rotate the catheter distal end by rotating its proximal end. Torqueability is useful, for example, in using a helical fixation member to temporarily or permanently secure a catheter to tissue. Flexibility, particularly along a distal end region of the catheter, becomes increasingly important as the catheter enters more serpentine or tortuous passages. Another characteristic that becomes more important with increased curvature of passages is the capacity to resist kinking, sometimes referred to as "kinkability".

The quest to provide treatment options for narrower vessels and other lumens has given rise to the need to reduce catheter sizes, yet retain their favorable structural properties. One successful approach involves providing a metal reinforcement layer within a polymeric catheter body. While several of the aforementioned patents concern this approach, reference also is made to U.S. Patent No. 5,836,926 (Peterson, et al.), disclosing an intravascular catheter that incorporates a reinforcing layer of interbraided stainless steel wires. The reinforcing layer is situated between an inside layer of polytetrafluoroethylene, and an outer layer comprising a blend of polyetherester elastomer and polybutylene terepthalate. As compared to a similarly sized catheter without the metallic reinforcement, this catheter can achieve improved pushability and torqueability, as well as improved kink resistance, while remaining sufficiently flexible.

In catheters used to deliver stents or other prostheses (e.g., grafts), a reduction in catheter diameter limits the prostheses deliverable by the device to smaller diameter prostheses, or alternatively increases the degree of radial compression applied to a prosthesis to enable its delivery. For a radially self-expanding prosthesis, the increased radial compression also increases the radial force between the prosthesis and surrounding catheter when the prosthesis is in its reduced-radius delivery state. The increase in radial force causes a proportionate increase in the frictional force that interferes with deployment of the prosthesis after its delivery to the treatment site.

To achieve further reductions in diameter of a metallic-braid-reinforced catheter, the thickness of the polymeric wall might be reduced, either with or without reducing the size of the wires or strands of the metal reinforcement. However, reducing the diameters of the strands reduces their strength, and thus diminishes one or more of the properties associated with strength: pushability, torqueability and kink resistance. Reducing the polymeric wall thickness while maintaining the strand size, however, eventually leads to unwanted exposure of the reinforcing braid.

Therefore, it is an object of the present invention to provide a catheter or other tubular medical device with a structure that, despite a reduction in diameter, maintains favorable properties related to the strength of a reinforcing strand. Another object is to provide a catheter structure that affords enhanced capacity to control desired properties.

A further object is to provide a process for fabricating a catheter that either enhances desired properties, or retains desired properties at acceptable levels despite a reduced diameter.

Yet another object is to provide a prosthesis delivery catheter capable of accommodating larger prostheses, or that requires less radial compression of a prosthesis during its delivery to a designated treatment site within a body lumen.

### Summary of the Invention

To achieve these and other objects, there is provided a body insertable prosthesis and delivery system, including:
a pliable tubular body having an inside surface, an outside surface, and a wall thickness in a radial direction, the tubular body surrounding an interior lumen;
a reinforcing structure including at least one elongate flexible structural strand integral with the tubular body and having a substantially uniform non-circular transverse profile with a thickness dimension and a width dimension at least twice the thickness dimension, wherein the at least one structural strand is disposed in a substantially surrounding relation to the interior lumen and oriented with the thickness dimension in the radial direction;
characterized to include a radially expandable prosthesis releasably contained in the interior lumen along a distal end region of the tubular body; and
a prosthesis release component disposed in the interior lumen and in contact with the prosthesis), said prosthesis release component and tubular body being adapted for axial travel relative to one another and operable to effect said axial travel;
further characterized in that the prosthesis when so contained further is in contact with the tubular body along the inside surface, and in that the inside surface is adapted to provide low-friction contact with the prosthesis whereby the tubular body and prosthesis release component, when operated to effect said axial travel, change the axial position of the tubular body and the prosthesis relative to one another and thereby are operable to effect a release of the prosthesis from within the interior lumen.

Thus, in accordance with the present invention, catheters with metal reinforcement can be formed with reduced diameters, yet retain favorable pushability, torqueability and resistance to kinking. These characteristics, along with flexibility, can be more precisely tailored to meet varying needs. Catheters used to deploy stents and other prostheses can be fashioned to handle prostheses of larger sizes, or to require a less severe radial contraction of a radially self-expanding prosthesis to achieve its delivery state. Alternatively, a catheter can be fashioned with the same prosthesis delivery capacity as a conventional catheter, but with a reduced diameter.

### In the Drawings

For a further understanding of the above and other features and advantages, reference is made to the following detailed description and to the drawings, in which:
Figure 1 is a side elevation of a prosthesis delivery and deployment device constructed in accordance with the present invention;
Figure 2 is an enlarged elevation, partially sectioned to show further features of the device;
Figure 3 is a sectional view taken along the line 3-3 in Figure 1;
Figure 4 is a schematic view of the braid pattern in which structural strands reinforcing the device are arranged;
Figure 5 is an enlarged view of a structural stand transverse profile;
Figure 6 is a graph illustrating distal end flexibility of the device as a function of pic rate;
Figure 7 is a graph illustrating flexibility of a proximal region of the device as a function of pic rate;
Figures 8-13 schematically illustrate fabrication of the device (not within the scope of the appended claims);
Figures 14a-d illustrate alternative structural strand profiles
Figure 15 illustrates a braid pattern of an alternative embodiment device (not within the scope of the appended claims); and
Figure 16 is a sectioned view of the alternative embodiment structural strand arrangement (not within the scope of the appended claims).

### Detailed Description of the Preferred Embodiments

Turning now to the drawings, there is shown a device 16 for delivering a radially self-expanding prosthesis to a selected treatment site within a body cavity or body lumen, and for deploying the prosthesis, once it is positioned at the treatment site. The device includes an elongate, flexible outer catheter 18 having a tubular catheter wall 20. A radiopaque marker 22 is mounted to the catheter near its distal end 24.

Along its axial length, catheter wall 20 is divided into two regions: a distal region 26 and a proximal region 28. As indicated by the break, the full length of proximal region 28 is not shown. Typically, the proximal region is considerably longer than the distal region. A distal end 27 of the catheter is joined to a hub 29.

Distal region 28 extends from distal end 24 to a junction 30 between two slightly different polymeric materials employed in forming the catheter wall. Along the distal region, the catheter wall preferably is more flexible, to enhance the capability of the catheter to negotiate serpentine or tortuous vascular passages. Along proximal region 28, the catheter preferably is less flexible to provide enhanced axial and circumferential strength, for enhanced pushability and torqueability, respectively. The device also must exhibit resistance to kinking along its entire length.

Figure 2 shows device 16 in greater detail. Outer catheter 18 includes a catheter lumen 32 that runs substantially the entire catheter length. An inner catheter 34, contained in the lumen, is moveable axially relative to the outer catheter. Inner catheter 34 extends lengthwise substantially along the entire length of the outer catheter. A sleeve 36 surrounds the inner catheter along a distal portion of the catheter comparable to catheter distal region 26 in its axial length. A prosthesis, in particular a radially self-expanding stent 38, surrounds the inner catheter and sleeve along the distal portion of the inner catheter. Stent 38 in turn is surrounded by the distal region of the outer catheter, constrained by the outer catheter wall in a radially reduced, axially elongated state. Stent 38 is radially self-expanding, in that as the stent is removed from the outer catheter, it tends to shorten axially and expand radially to a normal or unstressed state in which the stent diameter is larger than the diameter of the outer catheter. Stent 38 is composed of oppositely directed helically wound filaments or wires that intersect one another. A radiopaque marker 40 is located along inner catheter 34, between the inner catheter and the sleeve.

As best seen in Figure 2, catheter wall 20 has a layered construction including an inner layer or liner 42 that extends for the length of the catheter. Liner 42 is formed of a material selected to provide high lubricity (low friction), e.g., polytetrafluoroethylene (PTFE). Polyethylene also may be used.

Surrounding the inner layer is a reinforcing braid formed of multiple flexible elongate strands or wires 44, helically wound about the liner and interbraided with one another to form multiple crossings or intersections. Strands 44 can be formed of stainless steel, e.g., 304V stainless steel having a tensile strength of about 338 ksi. Other high tensile strength materials can be used, e.g., a cobalt-based alloy sold under the brand name Elgiloy. The braid reinforcement extends along the entire length of device 16, although in certain applications it may be desirable to provide a distal end region that does not incorporate the braid.

An outer layer surrounds the reinforcement braid, and is provided in the form of a distal outer tube 46 and a proximal outer tube 48. Strands 44 are embedded within the catheter wall, such that the wall thickness is determined substantially by the combined thicknesses of liner 42 and outer tube 46. Likewise, the wall thickness along proximal region 28 is determined by the thicknesses of liner 42 and tube 48. Depending on the method of fabrication, strands 44 are embedded substantially equally in both the liner and the outer layer, or alternatively are embedded substantially within the outer layer. Outer layers 46 and 48 abut one another at junction 30, and are bonded to the inner liner. Consequently, the liner, outer layers and reinforcing braid are integral with one another.

Outer layers 46 and 48 are preferably formed of a polyester ether, e.g., a homopolymer sold under the brand name Amitel, or a polymeric amide such as a nylon copolymer sold under the brand name Pebax. Further, however, different polymers are advantageously used to provide different degrees of flexibility in distal region 26 and proximal region 28. In particular, outer layer 46 can be formed of a polyester or nylon having a 63D hardness, while proximal outer layer 48 is formed of a material having a higher hardness, and therefore reduced flexibility but increased torqueability, pushability and resistance to kinking.

In particular versions of device 16, namely a "6 French" device and a "7 French" device, the catheter wall thickness is about 0,1 - 0,125 mm (0.004-0.005 inches), including liner 42 of about 0,025 mm (0.001 inches) thick and outer layers 46 and 48 at a thickness of 0,075 - 8,1 mm (0.003-0.004 inches). Strands 44 have rectangular transverse profiles, with a thickness of 0,0175 mm ± 0,05 mm (0.0007 inches ± 0.002 inches) and a width of 0,075 ± 0,0075 mm (0.003 inches ± 0.0003 inches). The catheter wall 20 is reduced in thickness, largely due to a reduction in strand thickness to 0,0175 mm (0.0007 inches), as compared to a 0,075 0.003 "thickness" of a conventional 0,075 mm (0.003 inch) diameter strand or wire. The reduced strand thickness allows a reduced thickness in catheter wall 20 without encountering unwanted strand exposure. As a result, the 6 French version of the device has an outer diameter of 1,975 mm (0.0790 inches), with an inner diameter enlarged to 1,725 mm (0.0690 inches). The 7 French device outer and inner diameters are 2,3 mm (0.0920 inches) and 1,95 mm (0.0780 inches), respectively.

The permitted reduction in outer diameter, or the permitted increase in internal diameter, actually is more than a direct comparison of the rectangular and circular strand might suggest. First, the impact of the thickness reduction is doubled because the overall diameter incorporates two thicknesses of the catheter wall. A further doubling occurs when the crossings or intersections of strands are taken into account.

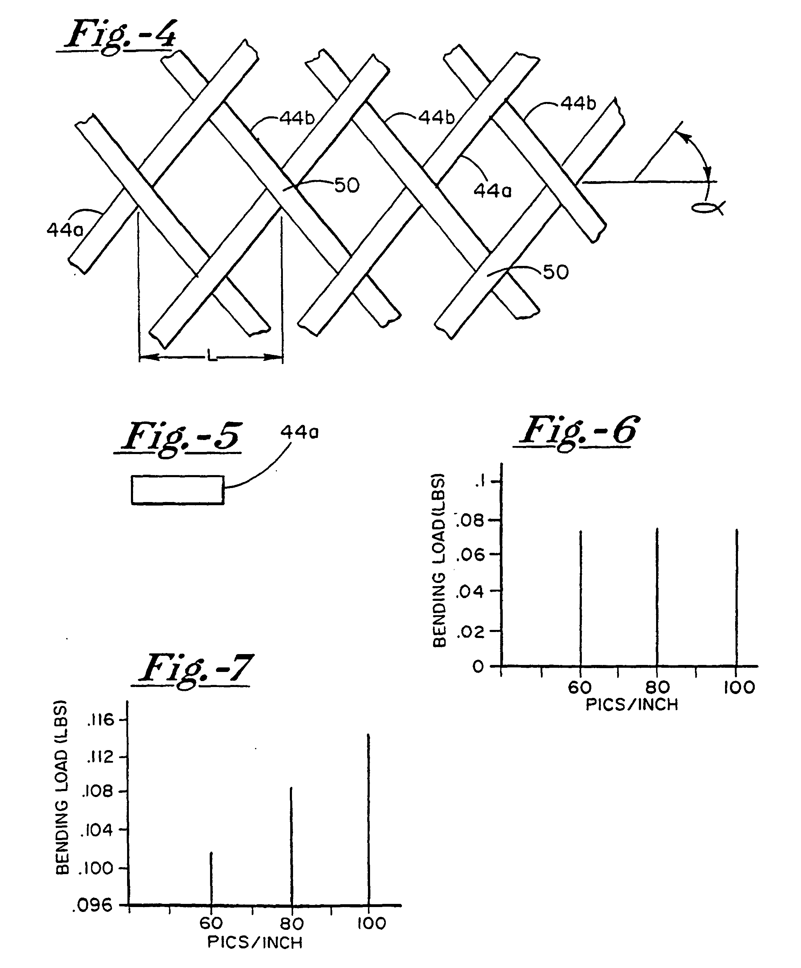

Figure 4 illustrates several strands 44a and 44b wound helically in respective first and second directions, and interbraided in a 1-over-1-under braid pattern. Alternate patterns, e.g., 2-over-2-under, can be used. In any event, the strands form multiple crossings or intersections 50, each involving one strand 44a and one strand 44b. The axial spacing between adjacent strands in both directions is substantially uniform. As a result, strands 44a and 44b cooperate to form multiple diamond or rhombic "pics" of substantially uniform size, particularly as to axial length L. The pic size also can be considered in terms of a rate of pics per inch. Specifically, a pic rate of 80-100 pics per inch determines a pic axial length range of 0,25 - 0,31 mm (0,010-0.0125 inches). The 80-100 pic rate has been found to provide satisfactory performance characteristics in both the 6 French and 7 French versions of the device.

Figure 5 shows an enlarged transverse profile of one of strands 44a. Strands 44b have substantially the same transverse profiles as to size and shape. As noted above, the rectangular profile affords the advantage of maintaining favorable catheter characteristics that depend on strand strength, yet reducing the required thickness of the catheter wall. While the advantage can be realized to an extent if the width is at least twice the thickness, more preferably the ratio of strand width dimension to strand thickness dimension is at least 3, more preferably in a range of 3.0-6.6.

Figure 6 is a graph illustrating the flexibility of distal region 26 in terms of the force required to bend the device, with a higher bending force representing reduced flexibility. The test was conducted with a self-expanding stent in the reduced-radius delivery state, maintained within interior lumen 32. The results indicate that varying the pic rate from 60 pics per inch to 100 pics per inch have negligible impact on flexibility.

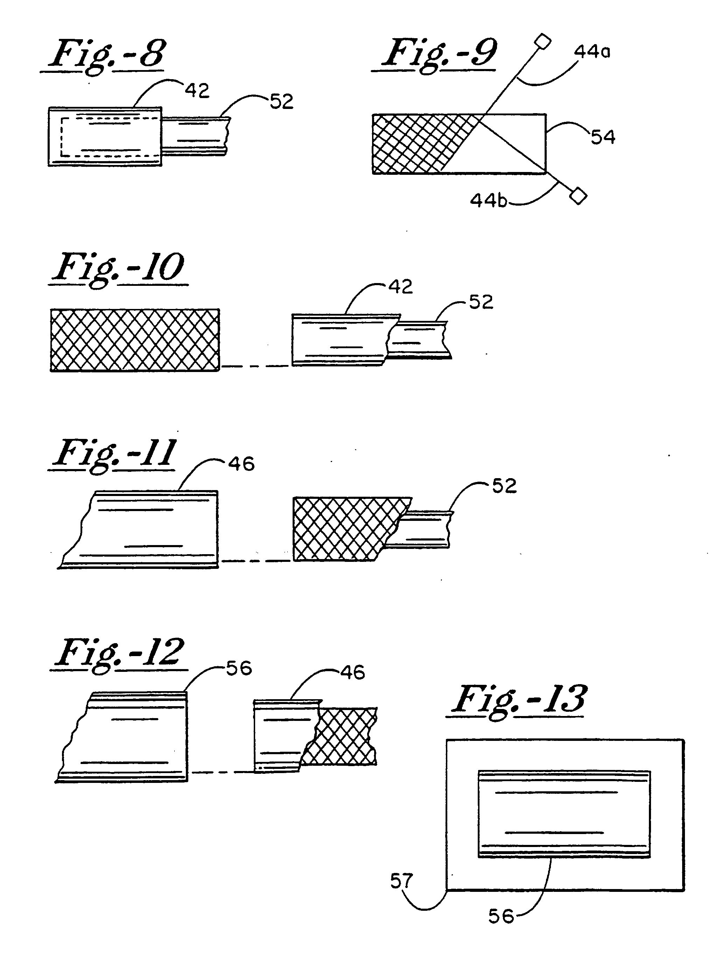

In contrast, varying the pic rate over the same range along proximal region 28 has a definite impact on flexibility, as indicated in the graph of Figure 7. In particular, the 6 French and 7 French versions of the device were found to have an undesirably high flexibility, and low kink resistance, when formed at a pic rate of 60 pics per inch. A pic rate of 80-100 pics per inch provided sufficient kink resistance while retaining sufficient flexibility. Further, flexibility was found to remain substantially stable, despite increases in the pic rate above 100 pics per inch, although this is not illustrated in Figure 7.

Figures 8-13 illustrate one approach to manufacturing device 16. As seen in Figure 8, an initial step involves placing a PTFE inside tube or liner 42 over an assembly mandrel 52. The assembly mandrel is selected based on the required inner diameter of the catheter. Next, strands 44 are wound onto a core rod 54 at a desired braid angle, axial spacing and braiding pattern. Adequate spring tension is placed on each braid spool to maintain the desired pattern. One preferred embodiment of the process involves interbraiding 16 strands, although the number of strands can be varied to suit different needs. The braider also is configured to provide a desired pic rate, e.g., 90 pics per inch.

After braiding, the reinforcement braided structure is removed from core rod 54 and placed around inside tube 42 on mandrel 52, as shown in Figure 10.

Next, an outside tube 46/48 is positioned around the braid (Figure 11). A heat shrink tube 56 is placed around the outside tube, to provide an assembly including the outside tubes, the reinforcement braid and the inside tube, along with the heat shrink tube, as seen in Figure 12. Heat is applied at a low rate, sufficient to cause the heat shrinkable tube 56 to shrink radially, thus to radially compress the remainder of the assembly. The radial compression of the assembly brings the outside tube and the inside tube more closely together, so that they are contiguous along an interface and cooperate to substantially surround or embed the reinforcing strands.

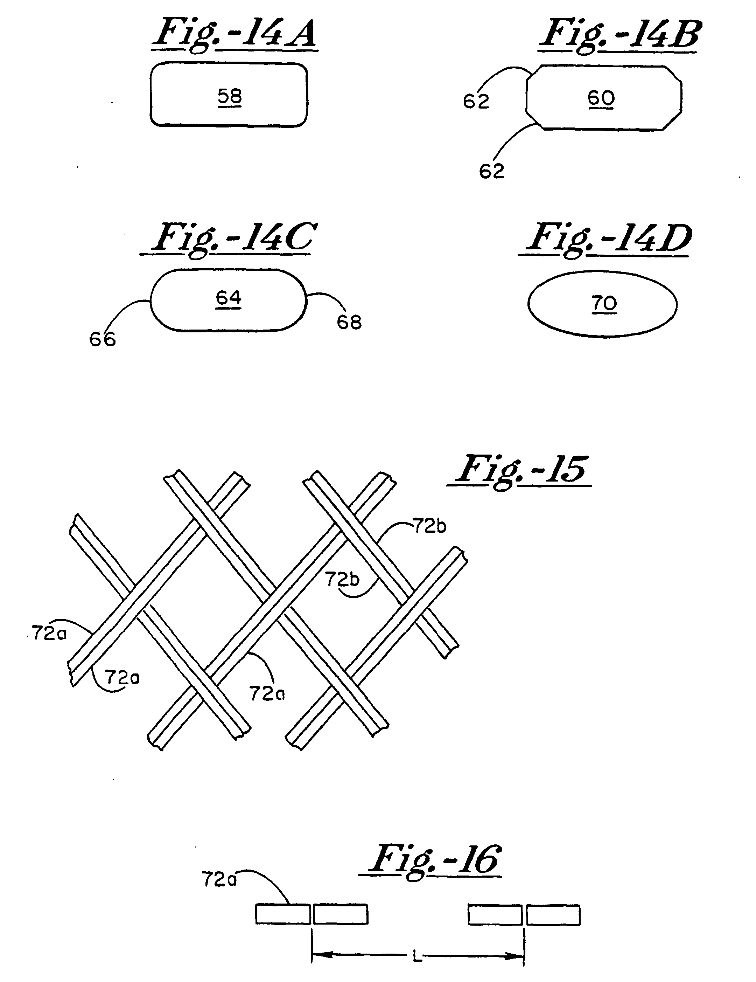

Finally, as seen in Figure 13, the radially compressed assembly is heated in a convection oven 57 at a considerably higher temperature (e.g., in the range of 300-500 degrees F, which causes outside tube 46/48 and inside tube 42 to form a fusion bond along their interface.

After the heat fusion and cooling of the assembly, the heat shrink tubing is cut and removed, yielding the completed catheter. In many cases there is a final step of etching a portion of exposed braid at the catheter distal end.

In an alternative fabrication process (not illustrated), the PTFE inside tube is placed over a copper core, after which the structural strands are braided onto the tube/core assembly. After braiding, an outer layer is applied by extrusion over and around the inside tube/braid assembly at a constant rate. The copper core shrinks when cooled, facilitating removal of the catheter.

The device has been found to exhibit satisfactory performance characteristics despite the reduced wall thickness enabled by the non-circular structural strands. For example, axial force (pushability) through a representative model was achieved by applying a force considerably less than the permitted maximum of 17 N (4.0 pounds) at 37 degrees C, while avoiding kinking of the device. Stent deployment and recapture forces likewise were considerably less than the required maximum allowable values. The tensile strength of the catheter/valve body connection exceeded the required minimum. Finally, kink resistance exceeded requirements. More particularly, with a device expected to pass through a 75 mm (3.0 inch) are successfully without kinking, the present devices were able to pass through a 50 mm (2.0 inch) radius arc, thus exceeding the requirement.

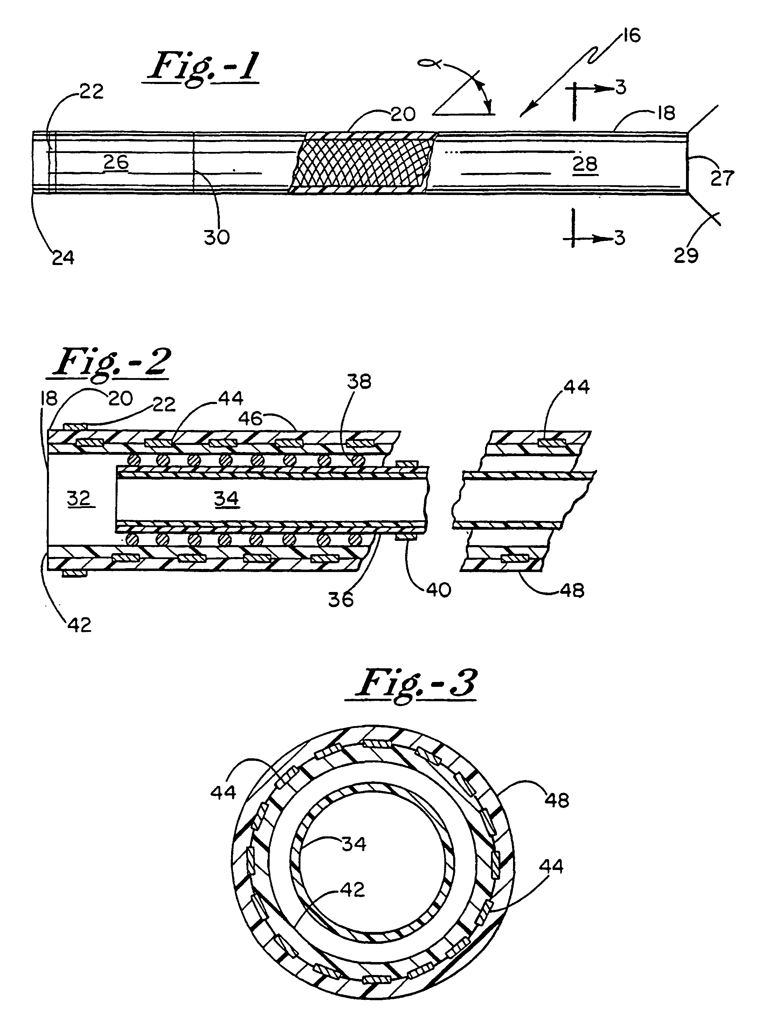

As noted above, a variety of factors can be selectively varied to obtain desired performance characteristics, including the strand width and thickness, the braid pattern, axial spacing between strands, and braid angle. The braid angle, illustrated at α in Figures 1 and 4, is the angle between the helical braid and the central axis of the braid, projected onto a plane containing the central axis, e.g., the plane of Figure 1. Increasing the braid angle tends to enhance torqueability, while reducing the braid angle tends to enhance pushability.

Figures 14a-d illustrate alternative embodiment structural strands with different transverse profiles, including a strand 58 with rounded corners, a strand 60 with beveled corners or edges 62, a strand 64 with rounded opposite sides 66 and 68, and a strand 70 with a substantially elliptical profile. In all cases, a width or major diameter preferably is in the range of 3.0-6.6 times the thickness or minor diameter.

Figures 15 and 16 illustrate an alternative embodiment reinforcing arrangement in which helically woven strands 72a and 72b are arranged in pairs of strands, with adjacent pairs spaced apart axially from one another. The distance "L" in Figure 16 illustrates the axial spacing between two pairs of strands, and thus also indicates an axial length of the pics formed in this alternative arrangement. Each strand 72a or 72b has a width of about 1.5 times its thickness. As a result, the side-by-side pair of strands has the given thickness, in combination with an effective width about three times the thickness. The strands could be arranged in sets of three or more side-by-side strands, if desired. Providing strands in sets can provide the aforementioned advantages, with an increase in flexibility as compared to the previous embodiment.

Thus, in accordance with the present invention, the outside diameter of a catheter can be reduced without a corresponding reduction in the diameter of its interior lumen, to facilitate use of the catheter in smaller diameter vascular passages and other body lumens. Alternatively, the size of a catheter's interior lumen may be increased without enlarging the catheter itself, thus to accommodate an increased fluid flow or a larger inner catheter or stent. Alternatively, the larger interior lumen can deliver a stent radially compressed to a lesser degree, reducing the radial elastic restoring force, thereby reducing friction to facilitate stent deployment.

## Claims

1. A body insertable prosthesis and delivery system, including:
a pliable tubular body (18) having an inside surface, an outside surface (20), and a wall thickness in a radial direction, the tubular body (18) surrounding an interior lumen (32);
a reinforcing structure including at least one elongate flexible structural strand (44) integral with the tubular body (18) and having a substantially uniform non-circular transverse profile with a thickness dimension and a width dimension at least twice the thickness dimension, wherein the at least one structural strand (44) is disposed in a substantially surrounding relation to the interior lumen (32) and oriented with the thickness dimension in the radial direction;
**characterized** to include a radially expandable prosthesis (38) releasably contained in the interior lumen (32) along a distal end region (26) of the tubular body (18); and
a prosthesis release component (34) disposed in the interior lumen (32) and in contact with the prosthesis (38), said prosthesis release component (34) and tubular body (18) being adapted for axial travel relative to one another and operable to effect said axial travel;
further **characterized in that** the prosthesis (38) when so contained further is in contact with the tubular body (18) along the inside surface, and **in that** the inside surface is adapted to provide low-friction contact with the prosthesis (38) whereby the tubular body (18) and prosthesis release component (34), when operated to effect said axial travel, change the axial position of the tubular body (18) and the prosthesis (38) relative to one another and thereby are operable to effect a release of the prosthesis (38) from within the interior lumen.

2. The system of claim 1 wherein:
the tubular body (18) comprises an inside layer (42) and an outside layer (46, 48) surrounding the inside layer (42), and the at least one structural strand (44) is disposed between the inside layer (42) and the outside layer (46, 48).

3. The system of claim 2 wherein:
the at least one structural strand (44) is substantially embedded in at least one of the inside layer (42) and the outside layer (46, 48).

4. The system of claim 2 wherein:
the outside layer (46, 48) is formed of a constituent selected from the group consisting of polyether esters and polymeric amides.

5. The system of claim 2 wherein:
said outside layer (46, 48) comprises a distal section (46) and a proximal section (48), and the outside layer is more flexible along the distal section (46) than along the proximal section (48).

6. The system of claim 1 wherein:
the prosthesis release component (34) includes an elongate, flexible member adapted to move the prosthesis (38) distally relative to the tubular body (18) to effect the release.

7. The system of claim 6 wherein:
the flexible member has a distal end portion surrounded by the prosthesis (38) when the prosthesis (38) is so retained.

8. The system of any of claims 1-7 wherein:
the prosthesis (38) is radially self-expanding, and when so retained is confined in a radially compressed state.

9. The system of claim 8 wherein:
the prosthesis (38) comprises a stent composed of oppositely directed helically wound filaments.

10. The system of claim 1 wherein:
the width dimension of the structural strand (44) is oriented normal to the radial direction.

11. The system of any of claims 1 to 10 wherein:
the tubular body (18) comprises a catheter, and the interior lumen (32) runs substantially the entire length of the catheter.

## Patentansprüche

1. In den Körper einführbare Prothese und Einführsystem, umfassend:
einen biegbaren, röhrenförmigen Körper (18) mit einer Innenfläche, einer Außenfläche (20) und einer Wanddicke in einer radialen Richtung, wobei der röhrenförmige Körper (18) einen inneren Hohlraum (32) umgibt;
eine Verstärkungskonstruktion, umfassend wenigstens einen länglichen, flexiblen Konstruktionsstrang (44), welcher mit dem röhrenförmigen Körper (18) integral ist und ein im Wesentlichen gleichförmiges, nicht kreisförmiges Querprofil aufweist, mit einem Dickenmaß und einem Breitenmaß, welches wenigstens das Doppelte des Dickenmaßes beträgt, wobei der wenigstens eine Konstruktionsstrang (44) im Wesentlichen umgebend bezüglich des inneren Hohlraums (32) angeordnet ist und mit dem Dickenmaß in radialer Richtung ausgerichtet ist;
**gekennzeichnet durch** Umfassen einer radial ausdehnbaren Prothese (38), welche freigebbar in dem inneren Hohlraum (32) entlang einem distalen Endbereich (26) des röhrenförmigen Körpers (18) aufgenommen ist; und
eine Prothesenfreigabekomponente (34), angeordnet in dem inneren Hohlraum (32) und in Berührung mit der Prothese (38), wobei die Prothesenfreigabekomponente (34) und der röhrenförmige Körper (18) für eine axiale Bewegung bezüglich einander ausgelegt sind und betreibbar sind, um diese axiale Bewegung auszuführen;
ferner **dadurch gekennzeichnet, dass** die derart aufgenommene Prothese (38) ferner entlang der Innenfläche in Berührung mit dem röhrenförmigen Körper (18) steht, und **dadurch**, dass die Innenfläche dafür ausgelegt ist, eine Niedrigreibungs-Berührung mit der Prothese (38) vorzusehen, wodurch der röhrenförmige Körper (18) und die Prothesenfreigabekomponente (34), wenn diese zum Ausführen der axialen Bewegung betrieben werden, die axiale Stellung des röhrenförmigen Körpers (18) und der Prothese (38) bezüglich einander verändern und **dadurch** zum Ausführen einer Freigabe der Prothese (38) aus dem Inneren des inneren Hohlraums betreibbar sind.

2. System nach Anspruch 1, wobei:
der röhrenförmige Körper (18) eine Innenschicht (42) und eine die Innenschicht (42) umgebende Außenschicht (46, 48) umfasst und der wenigstens eine Konstruktionsstrang (44) zwischen der Innenschicht (42) und der Außenschicht (46, 48) angeordnet ist.

3. System nach Anspruch 2, wobei:
der wenigstens eine Konstruktionsstrang (44) im Wesentlichen in wenigstens einer der Innenschicht (42) und der Außenschicht (46, 48) eingebettet ist.

4. System nach Anspruch 2, wobei:
die Außenschicht (46, 48) aus einem aus der Gruppe, umfassend Polyetherester und polymerische Amide, gewählten Bestandteil gebildet ist.

5. System nach Anspruch 2, wobei:
die Außenschicht (46, 48) einen distalen Abschnitt (46) und einen proximalen Abschnitt (48) umfasst und die Außenschicht entlang dem distalen Abschnitt (46) flexibler ist als entlang dem proximalen Abschnitt (48).

6. System nach Anspruch 1, wobei:
die Prothesenfreigabekomponente (34) ein längliches, flexibles Element umfasst, welches dafür ausgelegt ist, die Prothese (38) bezüglich des röhrenförmigen Körpers (18) distal zu bewegen, um die Freigabe auszuführen.

7. System nach Anspruch 6, wobei:
das flexible Element einen von der Prothese (38) umgebenen distalen Endabschnitt umfasst, wenn die Prothese (38) so gehalten ist.

8. System nach einem der Ansprüche 1-7, wobei:
die Prothese (38) radial selbstexpandierend ist und, wenn sie so gehalten ist, in einem radial komprimierten Zustand begrenzt ist.

9. System nach Anspruch 8, wobei:
die Prothese (38) einen Stent umfasst, welcher entgegengesetzt ausgerichtete, schraubenförmig gewundene Filamente aufweist.

10. System nach Anspruch 1, wobei:
das Breitenmaß des Konstruktionsstrangs (44) normal zu der Radialrichtung ausgerichtet ist.

11. System nach einem der Ansprüche 1 bis 10, wobei:
der röhrenförmige Körper (18) einen Katheter umfasst und der innere Hohlraum (32) im Wesentlichen über die gesamte Länge des Katheters verläuft.

## Revendications

1. Système de pose et de prothèse insérable dans le corps, comprenant :
un corps tubulaire pliable (18) présentant une surface intérieure, une surface extérieure (20), et une épaisseur de paroi dans une direction radiale, le corps tubulaire (18) entourant une lumière intérieure (32) ;
une structure de renfort comprenant au moins un fil structurel flexible allongé (44) formé d'un seul tenant avec le corps tubulaire (18) et présentant un profil transversal non circulaire sensiblement uniforme avec une dimension d'épaisseur et une dimension de largeur représentant au moins deux fois la dimension d'épaisseur, dans laquelle le au moins un fil structurel (44) est disposé sensiblement autour de la lumière intérieure (32) et orienté avec la dimension d'épaisseur dans la direction radiale ;
**caractérisé** pour comprendre une prothèse extensible radialement (38) contenue de manière amovible dans la lumière intérieure (32) le long d'une région d'extrémité distale (26) du corps tubulaire (18) ; et
un composant de libération de prothèse (34) disposé dans la lumière intérieure (32) et en contact avec la prothèse (38), ledit composant de libération de prothèse (34) et le corps tubulaire (18) étant adaptés pour une course axiale l'un par rapport à l'autre et actionnable pour effectuer ladite course axiale ;
**caractérisé en outre en ce que** la prothèse (38) lorsqu'elle est ainsi contenue est en contact avec le corps tubulaire (18) le long de la surface intérieure, et **en ce que** la surface intérieure est adaptée afin de conférer un contact à frottement réduit avec la prothèse (38) moyennant quoi le corps tubulaire (18) et le composant de libération de prothèse (34), lorsqu'ils sont actionnés afin d'effectuer ladite course axiale, modifient la position axiale du corps tubulaire (18) et de la prothèse (38) l'un par rapport à l'autre et sont ainsi actionnables afin d'effectuer une libération de la prothèse (38) de l'intérieur de la lumière intérieure.

2. Système selon la revendication 1, dans lequel :
le corps tubulaire (18) comprend une couche intérieure (42) et une couche extérieure (46, 48) entourant la couche intérieure (42), et le au moins un fil structurel (44) est disposé entre la couche intérieure (42) et la couche extérieure (46, 48).

3. Système selon la revendication 2, dans lequel :
le au moins un fil structurel (44) est sensiblement intégré dans au moins l'une de la couche intérieure (42) et de la couche extérieure (46, 48).

4. Système selon la revendication 2, dans lequel :
la couche extérieure (46, 48) est formée d'un composant choisi dans un groupe composé d'esters de polyéther et d'amides polymères.

5. Système selon la revendication 2, dans lequel :
ladite couche extérieure (46, 48) comprend une section distale (46) et une section proximale (48), et la couche extérieure est plus flexible le long de la section distale (46) que le long de la section proximale (48).

6. Système selon la revendication 1, dans lequel :
le composant de libération de prothèse (34) comprend un élément flexible allongé adapté pour déplacer la prothèse (38) de manière distale par rapport au corps tubulaire (18) pour effectuer la libération.

7. Système selon la revendication 6, dans lequel :
l'élément flexible présente une partie d'extrémité distale entourée par la prothèse (38) lorsque la prothèse (38) est ainsi retenue.

8. Système selon l'une quelconque des revendications 1 à 7, dans lequel :
la prothèse (38) est auto-extensible radialement, et lorsqu'elle est retenue ainsi est confinée dans un état comprimé radialement.

9. Système selon la revendication 8, dans lequel :
la prothèse (38) comprend un stent composé de filaments enroulés en hélice orientés de manière opposée.

10. Système selon la revendication 1, dans lequel :
la dimension de largeur du fil structurel (44) est orientée de manière perpendiculaire à la direction radiale.

11. Système selon l'une quelconque des revendications 1 à 10, dans lequel :
le corps tubulaire (18) comprend un cathéter, et la lumière intérieure (32) s'étend sensiblement sur toute la longueur du cathéter.
